Europäisches Patentamt

European Patent Office

Office européen des brevets

(19)

(11) Veröffentlichungsnummer: **0 205 675**
**A1**

(12)
# EUROPÄISCHE PATENTANMELDUNG

(21) Anmeldenummer: **85201621.1**

(51) Int. Cl.⁴: **A 61 K 7/04**

(22) Anmeldetag: **07.10.85**

(30) Priorität: **20.06.85 NL 8501770**

(43) Veröffentlichungstag der Anmeldung: **30.12.86**
**Patentblatt 86/52**

(84) Benannte Vertragsstaaten: **AT BE CH DE FR GB IT LI LU NL SE**

(71) Anmelder: **van Kleef, Hendrik, Westlander 139, NL-2636 DZ Schipluiden (NL)**

(72) Erfinder: **van Kleef, Hendrik, Westlander 139, NL-2636 DZ Schipluiden (NL)**

(54) **Kosmetisches Mittel zu fusshautheilender Wirkung und ein Verfahren zu deren Erzielung.**

(57) Die Erfindung ist ein kosmetisches und aktives Mittel, nicht homogenisiert worden mit Trägern. Das Verfahren ist direkt und fördert persönliche Hygiene an den Füßen, es stoppt Fußhauttranspiration und Geruch. Die Behandlung ist von kurzer Dauer und meistens einmalig. Wenn nach Jahren Fußtranspiration zurückkommt, dieselbe Behandlung kurz wiederholen. Die Behandlungsmethode ist von mir 35 Jahre erprobt worden.

Borsäure ($H_3-BO_3$) ist ein kosmetisches Desinfiziermittel wodurch die Fußhautsohle gegerbt wird und Nässe der Füße nicht mehr entsteht. Es wird gebracht in einer Packung mit Wickel auf dem eine Gebrauchsvorschrift angebracht worden ist.

Kosmetisches Mittel zu fußhautheilender Wirkung und ein Verfahren zu deren Erzielung.

Die Erfindung bezieht sich auf ein kosmetisches Mittel zu Fußhautheilender Wirkung, ein aktives Mittel umfassend, das nicht homogenisiert wird mit zur Herstellung kosmetischer Mittel üblichen Trägern, Verdünnungsmitteln und/oder Additiven. Die Erfindung bezieht sich auf ein direktes Verfahren zur Behandlung.

Allerhand kosmetische Mittel zur Fußhautbehandlung und persönlicher Hygiene sind bekannt, im allgemeinen ein natürlich vorhandenes aktives Mittel, hauptsächlich pflanzlichen Ursprungs-enthaltend, vermischt mit allerhand Trägern, Verdünnungsmitteln und anderen Additiven. Von Trägern wie Verdünnungsmittel und andere Additiven können z.B. Vaselin, tierische und pflanzliche Fette, raffinierter Talg, gebleichter Wachs und Bienenwachs, Konservierungsmittel, Parfüms usw. genannt werden.

Die Mittel, sogenannte Schuherfrischungsmittel, alle dieselbe Uebereinstimmung, sind so zahlreich, daß es unmöglich ist, hier eine vollständige Aufzählung zu geben. Von der Quelle der gemeinten aktiven Mittel werden hier nur als Beispiel Bama des-soft gestellt. (Deutsche Patentschrift 30737) Art. 33 genannt. Die Beschreibung in der holländischen Sprache ist keine Garantie, daß die Hauttranspiration wiederhergestellt werden kann. Die dreifache Wirkung: Stoppt Fußgeruch und hält Füße und Strümpfe angenehm frisch; Mild an den Füßen und verbessert die Luftzirkulation; Verhindert Fußschimmel und Fußgeruch, ist von kurzer Dauer. Schweißfeuchtigkeit aus der Fußhaut ist bleibend. Erfahrung mit Schuherfrischungsmitteln jeder Art ergeben ein entgegengestelltes Bild. Sie bestehen aus zwei verleimten dünnen Sohlen als Einlage im Schuh. Dadurch,daß die Untersohle perforiert worden ist, können Bakterien darin eine Brutstätte machen. Schuherfrischungssohlen verfaulen in sehr kurzer Zeit.

Ein nicht-zusammengesetztes aber zu ausschließlicher Fußhautbehandlung und dazu persönlicher Hygiene mit effektiver Wirkung, die Fußhauttranspiration und Gerüche völlig wiederherstellen kann, ist bis auf heute nicht bekannt.

Die Erfindung bezweckt ein kosmetisches Mittel zu verschaffen für persönliche Hygiene, das im Ganzen die menschliche Fußhaut wiederherstellen kann.

*2*

Anwendungsforschung für den äußerlichen Gebrauch, von mir erprobt in praktischer Hinsicht, verhindert transpirierende Füße und infolgedessen Fußgeruch.

Nach einer Periode Behandlung zwischen vier und sieben Tagen ist die Fußhaut für Jahre frei von Schweißfeuchtigheit. Es kann keinen Fußgeruch mehr geben.

Die Behandlung beruht auf dem chemischen Prinzip, Bor, chemisches Element mit Symbol B. Bor kommt vor in Kristallen, hart, silbergrau mit verschiedenen Formen. Der holländische Name für Bor ist "Boer". Bor kommt von einer großen Anzahl Mineralien in vulkanischen Gebieten.

Borverbindungen gibt es in verschiedenen Verbindungen, auf die ich hier nicht eingehen will. Von vielen kann die Formel wiedergegeben werden.

Für die Behandlung ist wichtig Borsäure ($H_3BO_3$) oder $B(OH)3$ und bildet sich bei der Hydrolyse einer großen Anzahl Borverbindungen. Sie wird u.a. gebraucht als Zwischenprodukt in der Lederbereitung, in der Kosmetik und sie ist ein Desinfektionsmittel. In der Kosmetik wird sie, soweit mir bekannt, nur gebraucht als irgendein Mittel, verarbeitet zusammen mit anderen Stoffen zur Bereitung von in der Kosmetik gebräuchlichen Trägern.

Darin liegen die Meinungsunterschiede. Bei der Lederbereitung nämlich wird Borsäure $B(OH)_3$ gebraucht zur Gerbung. Leder wird gemacht aus Tierhäuten. $B(OH)_3$ ist zugleich ein Desinfektionsmittel und als solches imstande Bakterien zu töten.

Das Ergebnis meiner 35 jährigen Erfahrung und Forschung ist folgendes:

Borsäure $B(OH)_3$ gerbt die Fußhaut bei Anwendung dieses Mittels im Schuh ohne schädliche Folgen, Fußtranspiration entsteht nicht mehr und die Fußhaut wird desinfiziert, Bakterien können nicht wirken und Transpirationsgerüche sind für lange Zeit, wenn nicht völlig, gebannt.

Die Methode der Behandlung habe ich vor 35 Jahren auf mich selbst angewandt.

Eine andere Versuchsperson, die das Mittel von mir bekommen hat, schreibt folgendes:

Unterzeichneter erklärt, daß er vor etwa 35 Jahren von Hendrik van Kleef ein Rezept erhalten hat zum beziehungsweise c.q. Verhindern von Fußtranspiration. Meine Erfahrung nach siebentägigem Gebrauch ist, daß ich nie wieder transpirierende Füße gehabt habe, dies trotz der Tatsache, daß ich vor der Behandlung die Socken ausringen konnte. Die Anwendung des Mittels hat für mich keine schädlichen Folgen gehabt.

Also der Briefschreiber

Beispiel I

Kosmetisch direktes Verfahren zur Fußhautbehandlung, mit Borsäure-Splittern $B(OH)_3$

Während der Behandlung:

1 abends Füße waschen mit kaltem Wasser vor dem Schlafengehen und Socken in die Wäsche geben.

2 streue auf die Fußsohle im Schuh einen Teelöffel Borsäure-Splitter, ist 1,43 Gramm.

3 morgens mit sauberen Socken Schuhe anziehen mit darin den unter 2 erwähnten Splittern.

4 dies etwa fünf bis sieben Tage wiederholen bis Füße nicht mehr transpirieren.

Beispiel II

Kosmetisch direktes Verfahren zur Fußhautbehandlung, mit Borsäure-Kristallen $B(OH)_3$

Eine gleiche Behandlung wie unter Beispiel I

Beispiel III

Kosmetisch direktes Verfahren zur Fußhautbehandlung, mit Borsäure-Puder $B(OH)_3$

Eine gleiche Behandlung wie unter Beispiel I und II

0205675

*CLAIMS*

## Schlußfolgerungen

1. Verfahren des kosmetischen Mittels zu FuBhautheilender Wirkung, Borsäure $H_3-BO_3$ ist kennzeichnend, nicht homogenisiert worden mit für kosmetik gebräuchlichen Trägern, Verdünnungsmitteln und/oder Additiven.

2. Das Verfahren nach Beispielen I bis einschlieBlich III, ist gleich, die FuBhaut kommt nicht in Berührung mit Borsäure $H_3-BO_3$.

## Kennzeichen

3. Borsäure $H_3-BO_3$. Farbe silbergrau ist ein rein kosmetisches fuBhautheilendes Mittel, enthält vulkanische Mineralien, desinfiziert, wodurch FuBsohlen gegerbt werden, geruchlos und hygienisch bleiben ohne schädliche Folgen, für lange Zeit, wenn nicht völlig wiederhergestellt. FuBhaut bleibt weich ohne Verschwielung.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch |
|---|---|---|
| X | FR-A-1 126 135 (PLANTIE)<br>* Seite 1, linke Spalte, Absatz 3 - rechte Spalte, Absatz 3; Zusammenfassung * | 1-3 |
| | --- | |
| X | FR-A-2 497 663 (GUISES)<br>* Seite 1, Zeilen 1-17; Patentanspruch * | 1,3 |
| | --- | |
| X | FR-A- 761 653 (HUET)<br>* Insgesamt * | 1,3 |
| | --- | |
| P,X | CHEMICAL ABSTRACTS, Band 103, 1985, Seite 307, Zusammenfassung Nr. 200709v, Columbus, Ohio, US; & NL - A - 85 01 770 (H. VAN KLEEF) 02.09.1985<br>* Insgesamt * | 1-3 |
| | ----- | |

**KLASSIFIKATION DER ANMELDUNG (Int. Cl.4)**

A 61 K    7/04

**RECHERCHIERTE SACHGEBIETE (Int. Cl.4)**

A 61 K    7/00

Der vorliegende Recherchenbericht wurde für alle Patentansprüche erstellt.

| Recherchenort | Abschlußdatum der Recherche | Prüfer |
|---|---|---|
| DEN HAAG | 25-04-1986 | WILLEKENS G.E.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82